(19) Europäisches Patentamt
European Patent Office
Office européen des brevets

(11) Veröffentlichungsnummer: **0 098 422**
**B1**

(12) # EUROPÄISCHE PATENTSCHRIFT

(45) Veröffentlichungstag der Patentschrift: **09.12.87**

(51) Int. Cl.⁴: **C 07 D 417/12** // (C07D417/12, 279:00, 213:00)

(21) Anmeldenummer: **83105888.8**

(22) Anmeldetag: **15.06.83**

(54) **Verfahren zur Herstellung von 4-Hydroxy-2-methyl-N-2-pyridyl-2H-1,2-benzothiazin-3-carboxamid 1,1-dioxid.**

(30) Priorität: **07.07.82 CH 4134/82**

(43) Veröffentlichungstag der Anmeldung: **18.01.84 Patentblatt 84/3**

(45) Bekanntmachung des Hinweises auf die Patenterteilung: **09.12.87 Patentblatt 87/50**

(84) Benannte Vertragsstaaten: **AT BE CH DE FR GB IT LI NL SE**

(56) Entgegenhaltungen:
**US - A - 3 591 584**
**US - A - 4 289 879**

**JOURNAL OF MEDICINAL CHEMISTRY, Band 16, Nr. 1, 1973; H. ZINNES et al. "1,2-Benzothiazines. 6. 3-Carbamoyl-4-hydroxy-2H-1,2-benzothiazine-1. 1-dioxides asantiinflammatory agents", Seiten 44-48**

**Die Akte enthält technische Angaben, die nach dem Eingang der Anmeldung eingereicht wurden und die nicht in dieser Patentschrift enthalten sind.**

(73) Patentinhaber: **SIEGFRIED AKTIENGESELLSCHAFT, CH-4800 Zofingen (CH)**

(72) Erfinder: **Zirngibl, Ludwig, Dr., Eisengrubenweg 16, CH-4800 Zofingen (CH)**
Erfinder: **Gnehm, René, Dr., Bornweg 8, CH-4665 Küngoldingen (CH)**

(74) Vertreter: **Jaeger, Klaus, Dipl.-Chem. Dr. et al, JAEGER & PARTNER Patentanwälte Pippinplatz 4a, D-8035 Gauting (DE)**

**EP 0 098 422 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

Die Erfindung betrifft ein Verfahren zur Herstellung von
4-Hydroxy-2-methyl-N-2-pyridyl-2H-1,2-benzothiazin-3-carboxamid 1,1 dioxid.

Dieses unter der INN-Bezeichnung Piroxicam bekanntgewordene Benzothiazin-Derivat hat in der Humantherapie als entzündungshemmendes Mittel Bedeutung erlangt.

Durch Amidierung der Carboxylgruppe erhaltene funktionelle Derivate von
2-Alkyl-4-hydroxy-2H-1,2-benzothiazin-3-carbonsäure 1,1-dioxiden
bzw. den damit tautomeren
2-Alkyl-3,4-dihydro-4-oxo-2H-1,2-benzothiazin-3-carbonsäure 1,1-dioxiden
und das zu solchen Carbamoylverbindungen führende Verfahren scheinen erstmals beschrieben worden zu sein in den auf die Priorität des US-Patentes 3 501 466 zurückgehenden Patentveröffentlichungen. Der bei jenem Verfahren beschrittene Weg führte vom N-(Ethoxycarbonyl)methyl-Derivat des Saccharins über eine ringerweiternde Umsetzung mit dem Ethoxid-Ion, Ammonolyse der dabei in 3-Stellung gebildeten Ethoxycarbonylgruppe und Alkylierung in 2-Stellung. In US-PS 3 591 584 wurde in der Folge eine grosse Zahl bisher nicht bekannter, in verschiedenen Positionen des Moleküls substituierter
4-Hydroxy-1,2-benzothiazin-3-carbonsäure 1,1 dioxide
bzw. entsprechender 3-Hydroxy-4-carbonsäure-Verbindungen beschrieben, darunter auch das eingangs genannte 2-Methyl-N-2-pyridyl-Derivat, d.h. das Piroxicam. Dabei wurden zwei mögliche Zugangswege zu diesen Verbindungen genannt, von denen der erste über die Umsetzung eines
4-Hydroxy- bzw. 3-Hydroxy-2H-1,2-benzothiazin 1,1-dioxids
mit einem Isocyanat und der andere wiederum über die Ammono- bzw. Aminolyse der in 3- oder 4-Position gebundenen Alkoxycarbonylgruppe verläuft, über eine Reaktion also, die sich für den Fall des eingangs genannten 2-Methyl-N-2-pyridyl-Derivates wie folgt darstellen lässt:

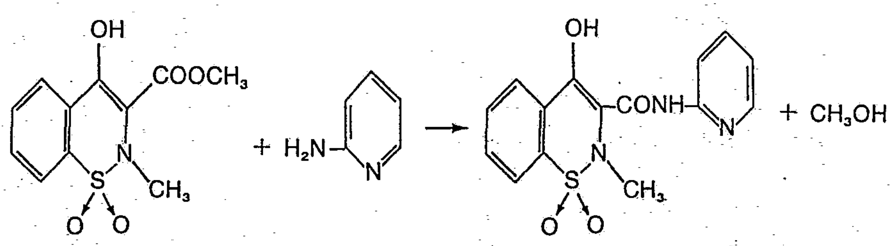

Für die Herstellung von 3-Carboxamiden mit heterocyclischem N-Substituenten drängt sich angesichts der Instabilität bzw. schwierigen Zugänglichkeit entsprechender Isocyanate der zweitgenannte Weg auf, obschon gerade das Aminolyse-Verfahren bei Durchführung in technischem Massstab ausgesprochen delikat ist. Zufolge der bekannten, zu Decarboxylierung führenden Instabilität der β-Keto-carbonsäure und der unter den Reaktionsbedingungen ebenfalls instabilen Natur des Aminopyridins bleibt man bei herkömmlicher Durchführung dieses Verfahrens auf Ausbeuten beschränkt, die nach Angaben der Literatur (J. Med. Chem. 15, 849 (1972) und 16, 493 (1973) sowie auch nach eigenen Untersuchungen bestenfalls 45% erreichen. Um aus dieser unbefriedigenden Situation herauszukommen, hat man verschiedene Wege beschritten. Zum einen hat man sich bemüht, durch einen gezielten Ringaufbau ausgehend von
N′-(2-Methoxycarbonylbenzolsulfonyl)-N′-methyl-N-2-pyridylglycinamid
direkt zum Endprodukt zu gelangen (US-PS 3 853 862 bzw. Re-issue 29 669), wobei man aber wieder des beim Aminolyse-Verfahren gegebenen Vorteils der leichten Zugänglichkeit des Vorproduktes verlustig geht. US-PS 3 891 637 beschreibt ein Verfahren, bei welchem das entsprechende 3-Carboxanilid (erhalten aus
4-Hydroxy-2H-1,2-benzothiazin 1,1-dioxid und Phenylisocyanat)
durch Umaminierung in das Pyridylderivat umgewandelt wird, wobei man aber eine weitere Reaktionsstufe mit zusätzlichem Ausbeuteverlust in Kauf zu nehmen hat.

US-PS 3 892 740 und 4 100 347 beschreiben schliesslich Verfahren, bei denen die 3-Alkoxycarbonylgruppe entweder nach intermediärer Umwandlung des in 4-Stellung befindlichen Keto-Sauerstoffs bzw. der 4-Hydroxygruppe in eine Alkoxygruppe oder unter Einhaltung spezieller Bedingungen zur 3-Carboxygruppe verseift und dann nach Umwandlung in ein reaktives Derivat wie beispielsweise in das Säurechlorid bzw. mittels eines Kupplungspromotors zum Pyridylcarboxamid amidiert wird, wobei aber wiederum zusätzliche Verfahrensstufen in Kauf genommen werden müssen und die Ausbeute (die nach Angaben der letztgenannten Schrift 30% betragen soll) offensichtlich nicht verbessert wird.

Überraschenderweise hat sich nun gezeigt, dass das bevorzugte, weiter oben erwähnte Aminolyse-Verfahren durch einfache Massnahmen so verbessert werden kann, dass die Ausbeute die Grössenordnung von 75 bis 80% annimmt, ohne dass dieser Zuwachs durch zusätzliche Verfahrensstufen erkauft werden muss. Es konnte nämlich festgestellt werden, dass sowohl die Ausgangsstoffe als auch das Produkt im Reaktionsgemisch in entscheidendem Ausmass stabilisiert werden durch die Anwesenheit einer Sulfonsäure, sofern diese in einem Anteil von mindestens 0,1

Mol Äquivalent, bezogen auf die Menge des mit dem Aminopyridin umzusetzenden Methylcarboxylats, zugegeben wird.

Die Erfindung besteht demnach in einem Verfahren zur Herstellung von
4-Hydroxy-2-methyl-N-2-pyridyl-2H-1,2-benzothiazin-3-carboxamid 1,1-dioxid
durch Umsetzung von
Methyl 4-hydroxy-2-methyl-2H-1,2-benzothiazin-3-carboxilat 1,1-dioxid
mit 2-Aminopyridin, welches dadurch gekennzeichnet ist, dass die Reaktion in Gegenwart einer Alkyl- oder Arylsulfonsäure in einer Menge von 0,1 bis 1,5 Mol-Äquivalent, bezogen auf die Menge des Methoxycarbonyl-Ausgangsstoffes, durchgeführt wird.

Beispiele von hiefür geeigneten Sulfonsäuren sind die Methan-, die Ethan-, die Benzol- und die p-Toluolsulfonsäure, und als besonders wirksam erwiesen sich Sulfonsäuremengen von 0,3 bis 0,7 Mol Äquivalent bezogen auf die Methoxycarbonylverbindung. Parallelversuche unter im übrigen gleichen Bedingungen ergaben ohne Sulfonsäure 39% Produkt-Ausbeute, mit 0,25 Äquivalent-Methansulfonsäure eine Ausbeute von 68%, bei 1,5 Äquivalent dagegen mit 62% eine Ausbeute, die bereits wieder deutlich unterhalb des erreichbaren Maximums lag.

Stabilisierender Effekt und Ausbeute können überdies vorteilhaft beeinflusst werden, wenn die Sulfonsäure-Zugabe portionenweise oder kontinuierlich über die ganze Dauer des Reaktionsablaufes erfolgt.

Hinweise auf die Zweckmässigkeit einer Sulfonsäure-Zugabe bei einer vergleichbaren Aminolyse-Reaktion finden sich zwar schon in der eingangs zitierten US-Patentschrift 3 591 584. Als Beispiel XXI wird dort die Umsetzung von 20,2 g
Methyl 4-hydroxy-2-methyl-2H-1,2-benzothiazin-3-carboxilat 1,1-dioxid
mit 14 g Anilin zum entsprechenden 3-Carboxanilid beschrieben, die in Anwesenheit von 25 Milligramm p-Toluolsulfonsäure durchgeführt wurde und eine Ausbeute von 35% lieferte. Zu beachten ist, dass in jenem Fall der Sulfonsäure-Anteil weniger als 0,002 Äquivalent des eingesetzten Esters ausmachte.

Weder dieser Veröffentlichung noch dem sonstigen Stand der Technik können irgenwelche Anhaltspunkte entnommen werden, aus denen sich hätte ableiten lassen, dass eine um einen Faktor von rund 250 erhöhte Sulfonsäure-Menge zu einer Ausbeute-Steigerung um einen Faktor von nahezu 2 führen könnte – im Gegenteil, empfiehlt doch die einschlägige Fachliteratur (z.B. J. Org. Chem. 28, 2915 (1963)), die Aminolyse von Carbonsäure-Estern in Gegenwart von basischen Katalysatoren durchzuführen, sodass denn auch in der Europäischen Patentveröffentlichung 3 360 auf Seite 11 angegeben wird, die Umsetzung von Estern der gemäss vorliegenden Erfindung benützten Art mit Aminen sei «erforderlichenfalls in Gegenwart eines basischen Kondensationsmittels» durchzuführen.

Die in Bezug auf den Mechanismus noch ungeklärte, günstige Beeinflussung der Aminolyse durch die erfindungsgemässe Verwendung einer relativ grossen Sulfonsäuremenge darf somit füglich als unvoraussehbar bezeichnet werden.

Die Umsetzung der 3-Methoxycarbonyl-Verbindung mit dem 2-Aminopyridin wird insbesondere in einem inerten organischen Lösungsmittel durchgeführt, und zwar vorzugsweise in einem niederen N,N-Dialkyl-alkanamid oder einem aromatischen Kohlenwasserstofflösungsmittel. Dabei haben die Alkyle im Alkanamid vorzugsweise bis zu vier Kohlenstoffatomen. Vorzugsweise wird die Reaktion in Dimethylformamid, Dimethylacetamid, Benzol, Toluol oder Xylol durchgeführt, wobei Xylol als Lösungsmittel speziell geeignet ist.

Die Reaktion wird vorzugsweise im Temperaturbereich zwischen Raumtemperatur und Siedetemperatur des Lösungsmittels, insbesondere im Stickstoffstrom, durchgeführt.

Zweckmässigerweise wird mit einem Überschuss an 2-Aminopyridin gearbeitet, vorzugsweise mit der zwei- bis dreifachen molaren Menge 2-Aminopyridin gegenüber der theoretisch erforderlichen äquimolaren Menge. Günstige Ergebnisse werden speziell dann erhalten, wenn zu Beginn zunächst nur ein Teil des insgesamt einzusetzenden 2-Aminopyridins vorgelegt wird und der Rest des insgesamt eingesetzten 2-Aminopyridins im Verlauf der Reaktion zugegeben wird, und zwar vorzugsweise in fester Form zugegeben wird.

Die Reaktion kann unter Rückfluss durchgeführt werden, erfolgt aber vorzugsweise unter ständigem langsamem Abdestillieren des Lösungsmittels.

Nach Abschluss der Reaktion wird der grösste Teil des verbliebenen Restes an Lösungsmittel rasch abdestilliert, und zwar vorzugsweise im lebhaften Stickstoffstrom, und wird dann das verbliebene Reaktionsgemisch abgekühlt und mit einer wässrigen Sulfonsäurelösung rasch versetzt oder in eine solche wässrige Sulfonsäurelösung eingeschüttet. Dabei wird vorzugsweise die gleiche Sulfonsäure verwendet, die auch bereits während der Reaktion zugesetzt worden ist. Anschliessend wird unter starkem Rühren weiter abgekühlt, insbesondere im Eiswasserbad, und das dabei ausgefallene Produkt anschliessend abfiltriert. Nachwaschen und Trocknen können erforderlichenfalls in üblicher Weise erfolgen.

Das Verfahren der Erfindung soll anhand des nachfolgenden Beispiels noch näher erläutert werden.

Beispiel

In einem 2,5 Liter Vierhalskolben, ausgestattet mit mechanischem Rührer, Innenthermometer, absteigendem Kühler mit durch ein Trockenrohr geschützter Vorlage und mit Tropftrichter, werden vorgelegt: 80,79 g (0,3 mol)
Methyl 4-hydroxy-2-methyl-2H-1,2-benzothiazin-3-carboxilat 1,1-dioxid,
42,34 g (0,45 mol) 2-Aminopyridin, 14,42 g (0,15 mol) Methansulfonsäure und 1,8 Liter Xylol (Isomerengemisch). Man erhitzt die Mischung unter Rühren im Ölbad und destilliert zunächst in ca. 1 Stunde etwa 150 ml Xylol ab, welches Spuren Feuchtigkeit enthält, und verwirft das Destillat. Dann wird die Badtemperatur so erniedrigt, dass weiteres Lösungsmittel sehr langsam (etwa 30–40 ml/Std.) übergeht. Dieses Destillat wird kontinuierlich durch Zutropfen von Xylol aus dem Tropftrichter ersetzt. Das Fortschreiten der Reaktion wird durch Dünnschicht-Chromatographie (Merck-Fertigplatten Kieselgel Nr. 5714, Laufmittel $CHCl_3$-Isopropanol 98:2) verfolgt. 7 Stunden nach Reaktionsbeginn gibt man in einer Portion 14,1 g (0,15 mol) 2-Aminopyridin in fester Form zu. 20 Stunden nach Reaktionsbeginn destilliert man ca. 800 ml Lösungsmittel schnell, d.h. innert ca. 1 Stunde ab, wobei man vorteilhafterweise im lebhaften Stickstoffstrom arbeitet.

Man kühlt die Reaktionsmischung sodann auf 60 °C Innentemperatur ab und lässt eine Lösung von 43,2 g (0,45 mol) Methanolsulfonsäure in 750 ml Wasser rasch zufliessen. Unter starkem Rühren kühlt man weiter im Eiswasserbad mindestens für 2 Stunden, dann wird abgesaugt, der Rückstand wird nachgewaschen und im Umluft-Trockenschrank mindestens 20 Stunden bei 55 °C getrocknet. Man erhält 77,0 g beiges Produkt, Smp. 191,5–193,5 °C, welches nach DC keines der Edukte mehr enthält. Ausbeute somit 0,232 mol oder 77,4% der Theorie.

**Patentansprüche**

1. Verfahren zur Herstellung von
4-Hydroxy-2-methyl-N-2-pyridyl-2H-1,2-benzothiazin-3-carboxamid 1,1-dioxid
durch Umsetzung von
Methyl 4-hydroxy-2-methyl-2H-1,2-benzothiazin-3-carboxilat 1,1-dioxid
mit 2-Aminopyridin, dadurch gekennzeichnet, dass die Reaktion in Gegenwart einer Alkyl- oder Arylsulfonsäure in einer Menge von 0,1 bis 1,5 Mol-Äquivalent, bezogen auf die Menge des Methoxycarbonyl-Ausgangsstoffes, durchgeführt wird.

2. Verfahren nach Anspruch 1, dadurch gekennzeichnet, dass man als Alkylsulfonsäure Methan- oder Ethansulfonsäure verwendet.

3. Verfahren nach Anspruch 1, dadurch gekennzeichnet, dass man als Arylsulfonsäure Benzolsulfonsäure oder eine Toluolsulfonsäure verwendet.

4. Verfahren nach Anspruch 1, dadurch gekennzeichnet, dass man die Sulfonsäure in einer Menge von 0,5 bis 1,0 Mol-Äquivalent bezogen auf die Methoxycarbonyl-Verbindung, verwendet.

5. Verfahren nach Anspruch 1, dadurch gekennzeichnet, dass die Sulfonsäure portionenweise verteilt über die ganze Dauer des Reaktionsablaufes zugegeben wird.

6. Verfahren nach Anspruch 1, dadurch gekennzeichnet, dass die Reaktion in einem inerten organischen Lösungsmittel im Temperaturbereich zwischen Raumtemperatur und Siedetemperatur des Lösungsmittels durchgeführt wird.

7. Verfahren nach Anspruch 6, dadurch gekennzeichnet, dass das inerte organische Lösungsmittel ein niederes N,N-Dialkyl-alkanamid, insbesondere Dimethylformamid oder Dimethylacetamid, oder ein aromatischer Kohlenwasserstoff, insbesondere Benzol, Toluol oder Xylol, ist.

8. Verfahren nach Anspruch 1, dadurch gekennzeichnet, dass nach Abschluss der Reaktion durch rasches Abdestillieren des Lösungsmittels das Reaktionsgemisch stark eingeengt und das Produkt durch rasche Zugabe von oder Eingiessen in eine wässrige Sulfonsäurelösung, insbesondere eine wässrige Lösung der Sulfonsäure, die während der Reaktion zugesetzt worden ist, ausgefällt wird.

9. Verfahren nach Anspruch 1, dadurch gekennzeichnet, dass die Reaktion unter Verwendung eines molaren Überschusses an 2-Aminopyridin, insbesondere der zwei- bis dreifachen molaren Menge 2-Aminopyridin, durchgeführt wird.

10. Verfahren nach Anspruch 5, dadurch gekennzeichnet, dass das 2-Aminopyridin in fester Form zugegeben wird.

**Claims**

1. A process for the preparation of
4-hydroxy-2-methyl-N-2-pyridyl-2H-1,2-benzothiazine-3-carboxamide 1,1-dioxide
by reacting
methyl 4-hydroxy-2-methyl-2H-1,2-benzothiazine-3-carboxylate 1,1-dioxide
with 2 aminopyridine, wherein the reaction is carried out in the presence of an alkyl- or arylsulfonic acid in an amount of 0.1 to 1.5 mole equivalent, based on the amount of the methoxycarbonyl starting material.

2. The process as claimed in claim 1, wherein methanesulfonic acid or ethanesulfonic acid is used as the alkylsulfonic acid.

3. The process as claimed in claim 1, wherein benzenesulfonic acid or a toluenesulfonic acid is used as the arylsulfonic acid.

4. The process as claimed in claim 1, wherein the sulfonic acid is used in an amount of 0.5 to 1.0 mole equivalent, based on the methoxycarbonyl compound.

5. The process as claimed in claim 1, wherein the sulfonic acid is added in portions, spread over the entire duration of the reaction.

6. The process as claimed in claim 1, wherein the reaction is carried out in an inert organic solvent in the temperature range between room temperature and the boiling point of the solvent.

7. The process as claimed in claim 6, wherein the inert organic solvent is a lower N,N-dialkyl alkanamide, in particular dimethylformamide or dimethylacetamide, or an aromatic hydrocarbon, in particular benzene, toluene or xylene.

8. The process as claimed in claim 1, wherein the reaction mixture is substantially evaporated down after the end of the reaction by rapidly distilling off the solvent, and the product is precipitated by rapidly adding an aqueous sulfonic acid solution or pouring the reaction mixture into an aqueous sulfonic acid solution, in particular an aqueous solution of the sulfonic acid which has been added during the reaction.

9. The process as claimed in claim 1, wherein the reaction is carried out using a molar excess of 2-aminopyridine, in particular a two-fold or threefold molar amount of 2-aminopyridine.

10. The process as claimed in claim 5, wherein the 2-aminopyridine is added in solid form.

**Revendications**

1. Procédé pour la préparation de
1,1-dioxyde de 4-hydroxy-2-méthyl-N-2-pyridyl-2H-1,2-benzothiazine-3-carboxamide
par réaction du
1,1-dioxyde de 4-hydroxy-2-méthyl-2H-1,2-benzothiazine-3-carboxylate de méthyle
sur la 2-aminopyridine, caractérisé par le fait que l'on conduit la réaction en présence d'un acide alcane- ou arènesulfonique en quantité de 0,1 à 1,5 équivalent molaire relativement à la quantité de la matière première méthoxycarbonyle.

2. Procédé selon la revendication 1, caractérisé en ce que comme acide alcanesulfonique on utilise l'acide méthane- ou éthanesulfonique.

3. Procédé selon la revendication 1, caractérisé en ce que comme acide arènesulfonique on utilise l'acide benzènesulfonique ou un acide toluènesulfonique.

4. Procédé selon la revendication 1, caractérisé en ce que l'on utilise l'acide sulfonique en une quantité de 0,5 à 1,0 équivalent molaire relativement au composé méthoxycarbonyle.

5. Procédé selon la revendication 1, caractérisé en ce que l'on ajoute l'acide sulfonique de façon répartie par portions sur toute la durée du déroulement de la réaction.

6. Procédé selon la revendication 1, caractérisé en ce que l'on conduit la réaction dans un solvant organique inerte, dans l'intervalle de température entre la température ambiante et la température d'ébullition du solvant.

7. Procédé selon la revendication 6, caractérisé en ce que le solvant organique inerte est une N,N-dialkyl-alcanamide inférieure, en particulier la diméthylacétamide, ou un hydrocarbure aromatique, en particulier le benzène, le toluène ou le xylène.

8. Procédé selon la revendication 1, caractérisé en ce qu'après la fin de la réaction, en chassant rapidement le solvant par distillation, on concentre fortement le mélange réactionnel et on le précipite en ajoutant rapidement une solution aqueuse d'acide sulfonique ou en le versant dans une telle solution, en particulier une solution aqueuse de l'acide sulfonique que l'on a ajoutée pendant la réaction.

9. Procédé selon la revendication 1, caractérisé en ce que l'on conduit la réaction avec utilisation d'un excès molaire de 2-aminopyridine, en particulier de deux à trois fois la quantité molaire de 2-aminopyridine.

10. Procédé selon la revendication 5, caractérisé en ce que l'on ajoute la 2-aminopyridine sous forme solide.